# EUROPÄISCHE PATENTSCHRIFT

(11) **EP 2 999 458 B1**
(45) Veröffentlichungstag und Bekanntmachung des Hinweises auf die Patenterteilung: **26.06.2019**
(21) Anmeldenummer: 14702051.5
(22) Anmeldetag: 03.02.2014
(51) Int. Cl.: A61K 8/42, A61Q 5/04, A61Q 5/08, A61Q 5/10, A61K 8/19, A61K 8/20, A61K 8/22, A61K 8/23

(54) **MINERALSALZE ZUR REDUZIERUNG DES CYSTEINSÄURE-GEHALTES IN KERATINISCHEN FASERN**
MINERAL SALTS FOR THE REDUCTION OF THE LEVEL OF CYSTEIC ACID IN THE KERATINIC FIBERS
SELS MINERAUX POUR REDUIRE LA QUANTITE D'ACIDE CYSTEIQUE DANS LES FIBRES DE KERATINE

(30) Priorität: 22.05.2013 DE 102013209498
(43) Veröffentlichungstag der Anmeldung: 30.03.2016
(73) Patentinhaber: Henkel AG & Co. KGaA, 40589 Düsseldorf (DE)
(72) Erfinder: GOUTSIS, Konstantin, 41363 Jüchen (DE); WESER, Gabriele, 41472 Neuss (DE)
(86) Internationale Anmeldenummer: PCT/EP2014/052010
(87) Internationale Veröffentlichungsnummer: WO 2014/187575

(56) Entgegenhaltungen:
- EP-A1- 2 417 964
- EP-A2- 2 143 419

## Beschreibung

Gegenstand der vorliegenden Anmeldung ist die kosmetische Verwendung von speziellen Mineralsalzen und deren Gemischen in Mitteln zur oxidativen Farbveränderung von keratinischen Fasern. Durch Einsatz der Mineralsalzgemische sollen die mit der Anwendung der Mittel verbundenen Schädigungen der keratinischen Fasern reduziert werden. Ein weiterer Gegenstand der vorliegenden Anmeldung sind Mittel zum oxidativen Farbveränderung von keratinischen Fasern, welche die Mineralsalzgemische enthalten. Diese Mittel eignen sich insbesondere zur Anwendung in einem Verfahren, bei welchem die Mittel wiederholt auf die keratinischen Fasern appliziert werden.

Die Veränderung der Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Farbveränderung der Haare kennt der Fachmann verschiedene Möglichkeiten. Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Die Färbung mit direktziehenden Farbstoffen ist mit einer geringen Haarschädigung verbunden, ein Nachteil ist jedoch die geringe Haltbarkeit und die schnelle Auswaschbarkeit der mit direktziehenden Farbstoffen erhaltenen Färbungen.

Wünscht sich der Verbraucher ein lang anhaltendes Farbergebnis oder eine Nuance, die heller als seine Ausgangshaarfarbe ist, werden üblicherweise oxidative Farbveränderungsmittel eingesetzt. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln untereinander die eigentlichen Farbstoffe ausbilden. Oxidationsfärbemittel zeichnen sich durch hervorragende, lang anhaltende Färbeergebnisse aus.

Als Oxidationsmittel wird in der Regel Wasserstoffperoxid eingesetzt, das nicht nur den Farbstoffbildungsprozess initiiert, sondern auch die haareigenen Melanin-Pigmente oxidativ zerstört. Hierdurch wird eine gleichzeitige Aufhellung des Haares erzielt. Die Einsatzmenge des Wasserstoffperoxids wird üblicherweise in Abhängigkeit vom gewünschten Aufhelleffekt gewählt. Oxidative Färbemittel enthalten in der Regel Mengen zwischen 3 und 12 Gew.-% Wasserstoffperoxid.

Die reine Aufhellung oder Blondierung von Haaren erfolgt oft durch Einsatz von Oxidationsmitteln ohne den Zusatz von Oxidationsfarbstoffvorprodukten. Für einen mittleren Blondiereffekt genügt der Einsatz von Wasserstoffperoxid als Oxidationsmittel allein, für das Erzielen eines stärkeren Blondiereffektes wird üblicherweise eine Mischung aus Wasserstoffperoxid und Peroxidisulfatsalzen eingesetzt.

Mit der Aufhellung geht jedoch zwangsläufig auch eine Schädigung des Haares einher, da nicht nur die Melanin-Pigmente, sondern auch die übrigen Strukturbestandteile des Haares oxidativ geschädigt werden. Die Melaninpigmente sind in Form von Granula in den Cortexzellen, d.h. im Inneren des Haares angeordnet. Mit der oxidativen Zerstörung der Melanin-Granula schädigt Wasserstoffperoxid nicht nur die äußere Schuppenschicht des Haares (Cuticula), sondern baut auch weitere, im Inneren der Haarfaser befindliche Strukturbestandteile oxidativ ab.

Die wichtigsten Bausteine der Haarfasern sind die Aminosäuren, die in den Proteinmolekülen der Keratinfasern durch Amidbindungen zu Makromolekülen verknüpft sind. Bedingt durch den Einfluss von Wasserstoffperoxid finden neben dem Melanin-Abbau auch an diesen Polypeptidketten chemische Abbaureaktionen statt, beispielsweise an den Cystin-Brücken, den Amid- und Aminogruppen sowie an den Wasserstoffbrücken und Salzbindungen der Peptidstruktur. Diese Abbau-Reaktionen führen zu starken Veränderungen der ursprünglichen mechanischen Eigenschaften des Haares.

Haarkeratine sind Proteine mit einem außergewöhnlich hohen Anteil (bis zu 10 Gew.-%) an Cystin. Durch das Cystin werden zwei Einheiten der Aminosäure Cystein, die sich in unterschiedlichen Peptidsträngen befinden, über eine Disulfid-Struktureinheit kovalent miteinander verknüpft. Die im Haar enthaltenen Cystine sind für die mechanische Stabilität der Haare von wesentlicher Bedeutung. Bei Einsatz von oxidativen Farbveränderungsmitteln wird nun ein Teil der in der Keratinfaser vorhandenen Cystin-Brücken oxidativ abgebaut. Hierbei werden die Disulfid-Gruppierungen des Cystins oxidativ gespalten und in Sulfonsäure-Einheiten überführt. Auf diese Weise wird das verschiedene Peptidstränge verknüpfende Cystin zu zwei diskreten, d.h. nicht mehr verbrückten, Cysteinsäure-Einheiten oxidiert.

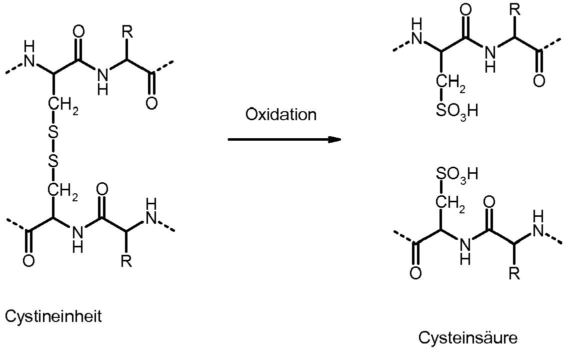

Aufgrund dieser Spaltungsreaktionen nimmt die Anzahl der Verknüpfungsstellen der Peptidstränge untereinander ab, und die mechanische Stabilität der Keratinfaser wird massiv beeinträchtigt. Je öfter die oxidativen Farbveränderungsmittel angewendet werden und je höher Wasserstoffperoxidgehalt (entweder allein oder in Kombination mit weiteren Oxidationsmitteln) in den Mitteln ist, desto größer ist der Stabilitätsverlust innerhalb des Peptidgerüstes. Dies äußert sich insbesondere in einer verminderten Widerstandsfähigkeit und verminderten Reißfestigkeit der Keratinfasern. Zudem ist ihre Dehnungsfähigkeit- insbesondere in ihrem nassen Zustand - stark erhöht. All diese Begleiterscheinungen oxidativer Farbveränderungsmittel sind vom Verbraucher in höchster Weise unerwünscht.

Die Aufgabe der vorliegenden Erfindung war die Bereitstellung von oxidativen Farbänderungsmitteln mit ausgezeichneten Färbe- und Aufhellleistungen und verringerter Haarschädigung. Keratinische Fasern, die mit entsprechenden oxidativen Farbänderungsmitteln behandelt wurden, sollten sich durch einen verringerten Cysteinsäuregehalt auszeichnen sowie eine verbesserte mechanische Stabilität, eine höhere Reißfestigkeit und eine erminderte Dehnungsfähigkeit besitzen. Hierbei sollte der Cysteinsäuregehalt der keratinischen Fasern insbesondere auch noch nach einer Mehrfachapplikation der oxidativen Farbänderungsmittel reduziert sein.

Der Fachmann kennt verschiedene Pflege- und Konditionierwirkstoffe, deren Einsatz in oxidativen Farbänderungsmitteln beschrieben ist. Bei diesen Pflegewirkstoffen handelt es sich üblicherweise um kationische Tenside oder kationische Polymere, die sich aufgrund ihrer positiven Ladung(en) an die negativ geladene Oberfläche (d.h. an die negativ geladene Cuticula) des Haares anlagern und auf diese Weise die Oberfläche der Fasern glätten bzw. konditionieren. Über diesen Mechanismus kann beispielsweise eine Verbesserung der Kämmbarkeit erreicht werden. Hierbei handelt es sich jedoch ausschließlich um Oberflächeneffekte, denn aufgrund ihrer verhältnismäßig hohen Molmasse und ihrer großen Molekülstruktur sind diese organischen Verbindungen nicht in der Lage, in das Innere der Haarfaser hinein zu diffundieren und dort direkt vor Ort die oxidative Degeneration des peptidischen Fasergerüstes zu verhindern. Auch bei den ebenfalls oft zur Konditionierung eingesetzten Ölen und polymeren Silikonverbindungen handelt es sich um analoge Oberflächeneffekte.

EP 2 417 964 A1 beschreibt ein Verfahren zur Reduzierung des Cysteinsäuregehaltes bei der chemischen Behandlung von Keratinfasern, bei welchem Diamide auf Basis von organischen Disäuren eingesetzt werden. Diese Disäuren können bis zu 20 C-Atome umfassen und stellen damit ebenfalls verhältnismäßig große organische Verbindungen dar.

Ein großer Nachteil dieser literaturbekannten organischen Konditionierwirkstoffe besteht hinsichtlich ihrer Akkumulation auf der Oberfläche der Keratinfasern, die sich insbesondere nach mehrfacher Anwendung der Mittel bemerkbar macht. Bei jeder Applikation lagern sich ein Teil der Pflegestoffe auf der Haaroberfläche ab. Bei entsprechend hoher Substantivität der Verbindungen zur Haaroberfläche (beispielsweise aufgrund ihrer positiven Ladung oder aufgrund ihrer Hydrophobizität) kann sich - vor allem, wenn der Konsument die Behandlung in sehr kurzen Zeitabständen wiederholt - ein Akkumulationseffekt ergeben, der zu beschwerten Haaren, geringem Frisurvolumen, fettigem Haargefühl (insbesondere bei Ölen und Silikonen) oder zu erhöhter statischer Aufladung, d.h. "Fliegen der Haare" (insbesondere bei Kationtensiden u. Kationpolymeren) führen kann. Die Folge ist eine "Überpflege" der Haare und ein unangenehmes Haargefühl. EP 2 143 419 A2 offenbart Oxidationsfärbemittel mit Gelbbasis aus hochethoxylierten Fettalkoholen und polysaccharidischem Verdicker für pH-reduzierte Expressfärbung. Als anorganische Verdicker sind beispielweise geeignete Eletrolyte, wie Natriumchlorid oder Kaliumchlorid. An keiner Stelle wird jedoch das spezifische Gewichtsverhältnis von (a) Natriumchlorid zu (d) Wasserstoffperoxid beschrieben.

Oxidative Farbänderungsmittel, die sich in unmittelbarer Weise positiv auf den Cysteinsäuregehalt der Haare auswirken, ohne gleichzeitig diese Nachteile aufzuweisen, sind aus dem Stand der Technik bislang nicht bekannt.

Überraschenderweise hat sich nun herausgestellt, das keratinische Fasern auch dann einen verringerten Cysteinsäuregehalt aufweisen, wenn sie mit oxidativen Farbänderungsmitteln behandelt werden, die als anorganisches Mineralsalz Natriumchlorid - entweder allein oder insbesondere im Gemisch mit anderen Mineralsalzen - enthalten. Die Mittel, welche diese Mineralsalzgemische enthalten, weisen die zuvor beschriebenen Nachteile nicht auf, d.h. die Mittel können auch in kurzen Zeitabständen mehrfach hintereinander appliziert werden, ohne dass eine Wirkstoffakkumulation auf der Oberfläche der Keratinfasern stattfindet.

Ein erster Gegenstand der vorliegenden Erfindung ist die kosmetische, nicht therapeutische Verwendung von
(a) Natriumchlorid
in Mitteln zum oxidativen Färben und/oder oxidativen Aufhellen von keratinischen Fasern zur Reduzierung der durch die Anwendung der Mittel entstandenen Schädigungen der keratinischen Fasern.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern geeignet sind, steht prinzipiell einer Verwendung auch auf anderen Gebieten nichts entgegen.

Unter der Reduzierung der durch die Anwendung der Mittel entstandenen Schädigungen der keratinischen Fasern wird im Sinne der vorliegenden Erfindung die Reduzierung der strukturellen Schädigungen des für die mechanische Festigkeit der Faser verantwortlichen Proteingerüstes verstanden. Eine bevorzugte Ausführungsform ist daher ist die kosmetische, nicht therapeutische Verwendung von (a) Natriumchlorid

in Mitteln zum oxidativen Färben und/oder oxidativen Aufhellen von keratinischen Fasern zur Reduzierung der durch die Anwendung der Mittel entstandenen strukturellen Schädigungen der keratinischen Fasern.

Eine besonders bevorzugte Ausführungsform ist die kosmetische, nicht therapeutische Verwendung von
(a) Natriumchlorid
in Mitteln zum oxidativen Färben und/oder oxidativen Aufhellen von keratinischen Fasern zur Reduzierung des Cysteinsäuregehaltes der mit diesen Mitteln behandelten keratinischen Fasern.

Der reduzierte Cysteinsäuregehalt der oxidativ behandelten keratinischen Fasern hat unmittelbare posititve Auswirkungen auf die mechanische Stabilität und die Reißfestigkeit der keratinischen Fasern.

Eine weitere bevorzugte Ausführungsform ist daher die kosmetische, nicht therapeutische Verwendung von
(a) Natriumchlorid
in Mitteln zum oxidativen Färben und/oder oxidativen Aufhellen von keratinischen Fasern zur Verbesserung der mechanischen Stabilität der mit diesen Mitteln behandelten keratinischen Fasern.

Eine weitere bevorzugte Ausführungsform ist auch die kosmetische, nicht therapeutische Verwendung von
(a) Natriumchlorid
in Mitteln zum oxidativen Färben und/oder oxidativen Aufhellen von keratinischen Fasern zur Verbesserung der Reißfestigkeit der mit diesen Mitteln behandelten keratinischen Fasern.

Bei Natriumchlorid (a) handelt es sich um die Verbindung NaCl, die auch Kochsalz oder Steinsalz genannt wird. Es kann Natriumchlorid verschiedensten Ursprungs eingesetzt werden. Das Natriumchlorid kann entweder in seiner kristallinen Form oder auch in Form einer Sole (wässrige NaCl-Lösung) eingesetzt werden. Auch der Einsatz von aus Meerwasser stammendem Natriumchlorid oder Meerwasser selbst ist möglich. Bevorzugt ist auch der Einsatz von Natriumchlorid in Abmischung mit anderen Mineralsalzen, wie es beispielsweise im Wasser von Mineralquellen vorkommt.

Ganz besonders bevorzugt wird Natriumchlorid aus der Mineralquelle von La Toja eingesetzt, entweder in Form des Wassers der Quelle oder in Form des Rückstandes, der beim Eindampfen des Quellwassers von La Toja entsteht.

Eine effektive Reduzierung des Cysteinsäuregehaltes konnte insbesondere auf keratinischen Fasern erzielt werden, die mit oxidativen Farbänderungsmitteln behandelt wurden, welche Natriumchlorid in Abmischung mit einem oder mehreren weiteren Mineralsalzen enthielten. Gute Messergebnisse wurden insbesondere dann erhalten, wenn die oxidativen Farbänderungsmittel ein Gemisch aus Natriumchlorid und Kaliumchlorid enthielten.

Eine ganz besonders bevorzugte Verwendung ist daher dadurch gekennzeichnet, dass die Mittel zusätzlich
(b) Kaliumchlorid
enthalten.

Eine besonders bevorzugte Ausführungsform ist damit die kosmetische, nicht therapeutische Verwendung von
(a) Natriumchlorid und
(b) Kaliumchlorid
in Mitteln zum oxidativen Färben und/oder oxidativen Aufhellen von keratinischen Fasern zur Reduzierung der durch die Anwendung der Mittel entstandenen Schädigungen der keratinischen Fasern.

Eine weitere besonders bevorzugte Ausführungsform ist die kosmetische, nicht therapeutische Verwendung von
(a) Natriumchlorid und
(b) Kaliumchlorid
in Mitteln zum oxidativen Färben und/oder oxidativen Aufhellen von keratinischen Fasern zur Reduzierung der durch die Anwendung der Mittel entstandenen strukturellen Schädigungen der keratinischen Fasern.

Eine weitere besonders bevorzugte Ausführungsform ist die kosmetische, nicht therapeutische Verwendung von
(a) Natriumchlorid und
(b) Kaliumchlorid
in Mitteln zum oxidativen Färben und/oder oxidativen Aufhellen von keratinischen Fasern zur Reduzierung des Cysteinsäuregehaltes der mit diesen Mitteln behandelten keratinischen Fasern.

Eine weitere bevorzugte Ausführungsform ist daher die kosmetische, nicht therapeutische Verwendung von
(a) Natriumchlorid und
(b) Kaliumchlorid
in Mitteln zum oxidativen Färben und/oder oxidativen Aufhellen von keratinischen Fasern zur Verbesserung der mechanischen Stabilität der mit diesen Mitteln behandelten keratinischen Fasern.

Eine weitere bevorzugte Ausführungsform ist auch die kosmetische, nicht therapeutische Verwendung von
(a) Natriumchlorid und
(b) Kaliumchlorid
in Mitteln zum oxidativen Färben und/oder oxidativen Aufhellen von keratinischen Fasern zur Verbesserung der Reißfestigkeit der mit diesen Mitteln behandelten keratinischen Fasern.

Oxidative Farbveränderungsmittel, welche Natriumchlorid (a) in bestimmten Mengenbereichen enthalten, haben sich als besonders wirksam zur Reduktion von oxidativen Schädigungen des Proteingerüstes der Faser herausgestellt. Die Verwendung der entsprechenden Mittel ist daher besonders bevorzugt.

Eine besonders bevorzugte Verwendung ist daher weiterhin dadurch gekennzeichnet, dass die Mittel Natriumchlorid (a) in einer Menge von 0,05 bis 3,5 Gew.-%, bevorzugt von 0,1 bis 2,5 Gew.-%, weiter bevorzugt von 0,2 bis 1,5 Gew.-% und besonders bevorzugt von 0,3 bis 0,8 Gew.-%, bezogen auf das Gesamtgewicht der anwendungsbereiten Mittel, enthalten.

Kaliumchlorid (b) wird in den oxidativen Farbveränderungsmitteln bevorzugt in einer Menge von 0,01 bis 1,0 Gew.-%, bevorzugt von 0,02 bis 0,5 Gew.-%, weiter bevorzugt von 0,03 bis 0,3 Gew.-% und besonders bevorzugt von 0,04 bis 0,1 Gew.-%, bezogen auf das Gesamtgewicht der anwendungsbereiten Mittel eingesetzt.

In einer weiteren besonders bevorzugten Ausführungsform ist eine erfindungsgemäße Verwendung daher dadurch gekennzeichnet, dass die Mittel Kaliumchlorid (b) in einer Menge von 0,01 bis 1,0 Gew.-%, bevorzugt von 0,02 bis 0,5 Gew.-%, weiter bevorzugt von 0,03 bis 0,3 Gew.-% und besonders bevorzugt von 0,04 bis 0,1 Gew.-%, bezogen auf das Gesamtgewicht der anwendungsbereiten Mittel, enthalten.

Ganz besonders bevorzugt ist die kosmetische, nicht therapeutische Verwendung von
(a) Natriumchlorid und
(b) Kaliumchlorid
in Mitteln zum oxidativen Färben und/oder oxidativen Aufhellen von keratinischen Fasern zur Reduzierung der durch die Anwendung der Mittel entstandenen Schädigungen der keratinischen Fasern, wobei die Mittel
- Natriumchlorid (a) in einer Menge von 0,05 bis 3,5 Gew.-%, bevorzugt von 0,1 bis 2,5 Gew.-%, weiter bevorzugt von 0,2 bis 1,5 Gew.-% und besonders bevorzugt von 0,3 bis 0,8 Gew.-%, bezogen auf das Gesamtgewicht der anwendungsbereiten Mittel, enthalten und
- Kaliumchlorid (b) in einer Menge von 0,01 bis 1,0 Gew.-%, bevorzugt von 0,02 bis 0,5 Gew.-%, weiter bevorzugt von 0,03 bis 0,3 Gew.-% und besonders bevorzugt von 0,04 bis 0,1 Gew.-%, bezogen auf das Gesamtgewicht der anwendungsbereiten Mittel, enthalten.

Als besonders vorteilhaft im Hinblick auf eine Verringerung der Haarschädigung hat es sich erwiesen, wenn Natriumchlorid (a) und Kaliumchlorid (b) als Gemisch in speziellen Gewichtsverhältnissen in den oxidativen Farbveränderungsmitteln eingesetzt werden. Von besonderem Vorteil ist in diesem Zusammenhang der Einsatz eines Überschusses an Natriumchlorid.

Ganz besonders bevorzugt ist daher auch eine Verwendung, welche dadurch gekennzeichnet ist, dass die Mittel Natriumchlorid (a) und Kaliumchlorid (b) in einem Gewichtsverhältnis (a)/(b) von 20:1 bis 1:1, bevorzugt von 18:1 bis 3:1, weiter bevorzugt von 16:1 bis 6:1 und besonders bevorzugt von 14:1 bis 9:1, bezogen auf das Gesamtgewicht der anwendungsbereiten Mittel, enthalten.

Demnach ebenfalls ganz besonders bevorzugt ist die kosmetische, nicht therapeutische Verwendung von
(a) Natriumchlorid und
(b) Kaliumchlorid
in Mitteln zum oxidativen Färben und/oder oxidativen Aufhellen von keratinischen Fasern zur Reduzierung der durch die Anwendung der Mittel entstandenen Schädigungen der keratinischen Fasern, wobei die Mittel
- Natriumchlorid (a) und Kaliumchlorid (b) in einem Gewichtsverhältnis (a)/(b) von 20:1 bis 1:1, bevorzugt von 18:1 bis 3:1, weiter bevorzugt von 16:1 bis 6:1 und besonders bevorzugt von 14:1 bis 9:1, bezogen auf das Gesamtgewicht der anwendungsbereiten Mittel, enthalten.

Zusätzlich zum Natriumchlorid (a) sowie gegebenenfalls Kaliumchlorid (b) können die oxidativen Farbänderungsmittel auch noch weitere anorganische Salze oder deren Gemische enthalten. Hierbei hat sich herausgestellt, dass der Zusatz eines oder mehrerer Salze aus der Gruppe Magnesiumcarbonat, Magnesiumhydrogencarbonat, Magnesiumsulfat, Magnesiumhydrogensulfat, Magnesiumchlorid, Calciumcarbonat, Calciumhydrogencarbonat, Calciumhydrogensulfat, Calciumsulfat und/oder Calciumchlorid den Cysteinsäureghelt und die Reißfestigkeit der keratinischen Fasern weiter erhöht. Damit ist auch eine Verwendung besonders bevorzugt, welche weiterhin dadurch gekennzeichnet ist, dass die Mittel zusätzlich mindestens ein weiteres Salz (c) aus der Gruppe Magnesiumcarbonat, Magnesiumhydrogencarbonat, Magnesiumsulfat, Magnesiumydrogenulfat, Magnesiumchlorid, Calciumcarbonat, Calciumhydrogencarbonat, Calciumhydrogenulfat, Calciumsulfat und/oder Calciumchlorid enthalten.

In anderen Worten ist damit die kosmetische, nicht therapeutische Verwendung von
(a) Natriumchlorid,
(b) Kaliumchlorid und
(c) mindestens einem weiteren Salz aus der Gruppe Magnesiumcarbonat, Magnesiumhydrogencarbonat, Magnesiumsulfat, Magnesiumydrogenulfat, Magnesiumchlorid, Calciumcarbonat, Calciumhydrogencarbonat, Calciumhydrogenulfat, Calciumsulfat und/oder Calciumchlorid
in Mitteln zum oxidativen Färben und/oder oxidativen Aufhellen von keratinischen Fasern zur Reduzierung der durch die Anwendung der Mittel entstandenen Schädigungen der keratinischen Fasern besonders bevorzugt.

Ebenfalls besonders bevorzugt ist die Verwendung des ersten Erfindungsgegenstands, welche dadurch gekennzeichnet ist, dass
- die Mittel mindestens eine Kombination aus zwei verschiedenen Salzen der Gruppe (c) enthalten, die ausgewählt ist aus Calciumchlorid/Magnesiumcarbonat, Calciumchlorid/Magnesiumhydrogencarbonat, Calciumchlorid/Magnesiumsulfat, Calciumchlorid/Magnesiumhydrogensulfat, Calciumchlorid/Magnesiumchlorid, Calciumchlorid/Calciumcarbonat, Calciumchlorid/Calciumhydrogencarbonat, Calciumchlorid/Calciumhydrogensulfat und/oder Calciumchlorid/Calciumsulfat.

In anderen Worten ist damit die kosmetische, nicht therapeutische Verwendung von
(a) Natriumchlorid,
(b) Kaliumchlorid und
(c) mindestens einer Kombination aus zwei verschiedenen Salzen aus der Gruppe Calciumchlorid/ Magnesiumcarbonat, Calciumchlorid/Magnesiumhydrogencarbonat, Calciumchlorid/Magnesiumsulfat, Calciumchlorid/Magnesiumhydrogensulfat, Calciumchlorid/Magnesiumchlorid, Calciumchlorid/Calciumcarbonat, Calciumchlorid/Calciumhydrogencarbonat, Calciumchlorid/Calciumhydrogensulfat und/oder Calciumchlorid/Calciumsulfat
in Mitteln zum oxidativen Färben und/oder oxidativen Aufhellen von keratinischen Fasern zur Reduzierung der durch die Anwendung der Mittel entstandenen Schädigungen der keratinischen Fasern besonders bevorzugt.

Mittel zum oxidativen Färben bzw. oxidativen Aufhellen von keratinischen Fasern enthalten zur Initiierung des Färbeprozesses sowie zur oxidativen Zerstörung der haareigenen Melanin-Pigmente (d.h. zur Erzeugung des Aufhelleffektes) mindestens ein Oxidationsmittel. Üblicherweise handelt es sich hierbei um Wasserstoffperoxid. Experimentell konnte im Zuge der zu dieser Erfindung führenden Arbeiten nachgewiesen werden, dass Natriumchlorid (a) - entweder allein oder in Kombination mit den zuvor beschriebenen weiteren Mineralsalzen - sich sehr gut eignet, um die durch Wasserstoffperoxid hervorgerufenen strukturellen Schädigungen der Keratinfasern zu verringern.

Eine weitere bevorzugte Verwendung ist daher dadurch gekennzeichnet, dass die Mittel als Oxidationsmittel (d) Wasserstoffperoxid enthalten.

Mit anderen Worten ist damit die kosmetische, nicht therapeutische Verwendung von
(a) Natriumchlorid
in Mitteln zum oxidativen Färben und/oder oxidativen Aufhellen von keratinischen Fasern zur Reduzierung der durch die Anwendung der Mittel entstandenen Schädigungen der keratinischen Fasern besonders bevorzugt, wobei die Mittel als Oxidationsmittel (d) Wasserstoffperoxid enthalten.

Es hat sich herausgestellt, dass Natriumchlorid (a) insbesondere dann die schädlichen Auswirkungen des Wasserstoffperoxids (d) auf die Peptidstruktur des Haares minimiert, wenn Natriumchlorid und Wasserstoffperoxid in bestimmten Mengenverhältnissen zueinander eingesetzt werden. Um den Cysteinsäuregehalt der Keratinfasern signifikant zu reduzieren, ist es von Vorteil, auf 10 Gewichtsanteile Wasserstofferperoxid (d) zumindest 1 Gewichtsanteil Natriumchlorid (a) einzusetzen.

Eine bevorzugte Verwendung ist daher weiterhin dadurch gekennzeichnet, das die Mittel Natriumchlorid (a) und Wasserstoffperoxid (d) in einem Gewichtsverhältnis (a)/(d) von 2:1 bis 1:10, bevorzugt von 1:1 bis 1:8, weiter bevorzugt von 1:2 bis 1:8 und besonders bevorzugt von 1:3 bis 1:6, bezogen auf das Gesamtgewicht der anwendungsbereiten Mittel, enthalten.

Mit anderen Worten ist damit die kosmetische, nicht therapeutische Verwendung von
(a) Natriumchlorid
in Mitteln zum oxidativen Färben und/oder oxidativen Aufhellen von keratinischen Fasern zur Reduzierung der durch die Anwendung der Mittel entstandenen Schädigungen der keratinischen Fasern besonders bevorzugt, wobei die Mittel Natriumchlorid (a) und Wasserstoffperoxid (d) in einem Gewichtsverhältnis (a)/(d) von 2:1 bis 1:10, bevorzugt von 1:1 bis 1:8, weiter bevorzugt von 1:2 bis 1:8 und besonders bevorzugt von 1:3 bis 1:6, bezogen auf das Gesamtgewicht der anwendungsbereiten Mittel, enthalten.

Der Begriff der oxidativen Farbveränderung umfasst sowohl die oxidative Färbung als auch die oxidative Aufhellung von keratinischen Fasern.

Die Mittel zum oxidativen Färben und/oder oxidativen Aufhellen von keratinischen Fasern enthalten die zuvor beschriebenen erfindungswesentlichen Inhaltsstoffe in einem kosmetischen Träger. Der kosmetische Träger ist bevorzugt wässrig, alkoholisch oder wässrig-alkoholisch. Für die Anwendung auf dem Haar geeignet sind beispielsweise Cremes, Emulsionen, Gele oder auch tensidhaltige schäumende Lösungen, wie beispielsweise Schaumaerosole oder schaumförmige Non-Aersosol-Applikationen. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen.

Bei dem in den oxidativen Farbänderungsmitteln enthaltenen Oxidationsmittel handelt es sich bevorzugt um Wasserstoffperoxid und/oder eines seiner festen Anlagerungsprodukte an organische oder anorganische Verbindungen.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,5 bis 20 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des Mittels, enthalten.

Oxidative Färbemittel enthalten neben dem Oxidationsmittel mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Zusätzlich können die oxidativen Färbemittel ebenfalls mindestens einen direktziehenden Farbstoff aus der Gruppe der anionischen, nichtionischen und/oder kationischen Farbstoffe enthalten.

Die Oxidationsfarbstoffvorprodukte, d.h. Entwicklerkomponenten und Kupplerkomponenten, sowie die otpional zusätzlich enthaltenen direktziehenden Farbstoffe werden bevorzugt jeweils in einer Menge von 0,0001 bis 5,0 Gew.-%, vorzugsweise 0,001 bis 3,5 Gew.-%, jeweils bezogen auf das Gesamtgewicht des anwendungsbereiten Mittels, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Mittel zum oxidativen Aufhellen (d.h. Blondieren) von keratinischen Fasern können zur Nuancierung ebenfalls in geringeren Mengen Oxidationsfarbtoffvorprodukte enthalten. In einer bevorzugten Ausführungsform sind diese jedoch frei von Oxidationsfarbstoffvorprodukten. Zur Erzielung eines stärkeren Aufhelleffektes können oxidative Aufhellmittel Wasserstoffperoxid in Kombination mit einem oder mehreren weiteren Oxidationsmitteln, insbesondere mit Peroxosalzen, enthalten.

Geeignete Peroxo-Salze sind anorganische Peroxoverbindungen, bevorzugt ausgewählt aus der Gruppe aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Die Persulfate sind jeweils in einer Menge von 0,5 bis 20 Gew.-%, vorzugsweise 1 bis 12,5 Gew.-%, besonders bevorzugt 2,5 bis 10 Gew.-% und insbesondere 3 bis 6 Gew.-%, bezogen auf das Gesamtgewicht des anwendungsbereiten Mittels, in den Mitteln zum oxidativen Aufhellen von keratinischen Fasern enthalten.

Wie bereits zuvor beschrieben sind oxidative Farbänderungsmittel, welche Natriumchlorid (a) in Kombination mit Kaliumchlorid (b) enthalten, besonders geeignet zur schädigungsarmen Färbung und Aufhellung von keratinischen Fasern. Die verminderte Schädigung äußert sich vor allem darin, dass die (insbesondere mehrfach) mit den Mitteln behandelten Fasern einen verringerten Cysteinsäuregehalt aufweisen.

Ein zweiter Gegenstand der vorliegenden Erfindung ist daher ein anwendungsbereites Mittel zum oxidativen Färben und/oder oxidativen Aufhellen von keratinischen Fasern, enthaltend (a) Natriumchlorid,
(b) Kaliumchlorid und
(d) Wasserstoffperoxid.

Durch den Einsatz mindestens eines weiteren Mineralsalzes aus einer bestimmten Gruppe kann der Cysteinsäuregehalt der behandelten Keratinfsern noch weiter reduziert werden.

Eine bevorzugte Ausführungsform ist daher ein anwendungsbereites Mittel zum oxidativen Färben und/oder oxidativen Aufhellen von keratinischen Fasern, enthaltend
(a) Natriumchlorid,
(b) Kaliumchlorid,
(c) gegebenenfalls mindestens ein weiteres Salz aus der Gruppe Magnesiumcarbonat, Magnesiumhydrogencarbonat, Magnesiumsulfat, Magnesiumhydrogensulfat, Magnesiumchlorid, Calciumcarbonat, Calciumhydrogencarbonat, Calciumhydrogensulfat, Calciumsulfat und/oder Calciumchlorid und
(d) Wasserstoffperoxid.

Eine besonders bevorzugte Ausführungsform ist ein anwendungsbereites Mittel zum oxidativen Färben und/oder oxidativen Aufhellen von keratinischen Fasern, enthaltend
(a) Natriumchlorid,
(b) Kaliumchlorid,
(c) mindestens ein weiteres Salz aus der Gruppe Magnesiumcarbonat, Magnesiumhydrogencarbonat, Magnesiumsulfat, Magnesiumhydrogensulfat, Magnesiumchlorid, Calciumcarbonat, Calciumhydrogencarbonat, Calciumhydrogensulfat, Calciumsulfat und/oder Calciumchlorid und
(d) Wasserstoffperoxid.

Oxidative Farbänderungsmittel werden in der Regel in der Form eines Zwei- oder Mehrkomponentenmittels konfektioniert. Hierbei enthält die erste Komponente (I) ein Alkalisierungsmittel sowie, falls zusätzlich zur Aufhellung auch eine Nuancierung oder Färbung durchgeführt werden soll, die Oxidationsfarbstoffvorprodukte. Die zweite Komponente (II) enthält das Oxidationsmittel bzw. das Oxidationsmittelgemisch. Das anwendungsbereite Mittel wird vom Verbraucher direkt vor der Anwendung durch Vermischen der beiden Komponenten (I) und (II) hergestellt.

Die das Alkalisierungsmittel (sowie ggf. die Oxidationsfarbstoffvorprodukte) enthaltende Komponente (I) und die Oxidationsmittel (d.h. Wasserstoffperoxid) enthaltende Komponente (II) werden in der Regel zu gleichen Gewichtsanteilen (d.h. im Gewichtsverhältnis 1:1) miteinander vermischt. Es können aber auch Gewichtsverhältnisse von 1:4 bis 4:1 (Alkalisierungsmittelkomponente: Oxidationsmittelkomponente), bevorzugt von 1:3 bis 3:1 und weiter bevorzugt von 1:2 bis 2:1 gewählt werden. Ganz besonders ist der Einsatz beider Komponenten im Gewichtsverhältnis 1:1.

Sofern im oxidativen Färbänderungsmittel Wirk- oder Inhaltsstoffe eingesetzt werden sollen, die weder mit Alkalisierungsmittel noch mit Oxidationsmitteln kompatibel sind, ist zur Erhöhung der Lagerstabilität auch die Konfektionierung in Form eines Dreikomponentenmittels denkbar. In diesem Fall wird das anwendungsbereite Mittel direkt vor der Anwendung durch den Verbraucher durch Vermischen einer Alkalisierungsmittelkomponente (welche gegebenenfalls zusätzlich Oxidationsfarbstoffvorprodukte enthält) (I), einer Oxidationsmittelkomponente (II) und der weiteren dritten Komponente (III) hergestellt. In jedem der beschriebenen Fälle wird unter dem anwendungsbereiten Mittel das Mittel verstanden, welches direkt vor der Anwendung durch Vermischen aller notwendigen Einzelkomponenten erhalten wurde und welches alle für den Aufhell- bzw. Färbevorgang wesentlichen Bestandteile (d.h. Alkalisierungsmittel, Oxidationsmittel und ggf. Oxidationsfarbstoffvorprodukte) enthält. Alle angegebenen Mengenangaben beziehen sich jeweils auf das Gesamtgewicht des anwendungsbereiten Mittels. Natriumchlorid (a) und Kaliumchlorid (b) sind sowohl gegenüber den Alkalisierungsmitteln als auch gegenüber den Oxidationsmitteln stabil. Die Mineralsalze können daher sowohl in die Komponente, welche das Alkalisierungsmittel enthält, als auch in die das Oxidationsmittel enthaltende Komponente eingearbeitet werden. In beiden Fällen zeichnen sich die Formulierungen durch eine hervorragende Lagerstabilität aus, die der Lagerstabilität entsprechender Formulierungen mit organischen Konditionier- und Pflegestoffen weit überlegen ist.

Zusammenfassend zeichnet sich eine weitere bevorzugte Ausführungsform dadurch aus, dass das anwendungsbereite oxidative Farbänderungsmittel durch Vermischen der zwei Komponenten (I) und (II) hergestellt wird, wobei
(I) die ersten Komponente mindestens ein Alkalisierungsmittel, gegebenenfalls Oxidationsfarbstoffvorprodukte, Natriumchlorid (a) und Kaliumchlorid (b) enthält und
(II) die zweite Komponente Wasserstoffperoxid enthält.

Die erfindungsgemäßen Mineralsalze weisen auch gegenüber Oxidationsmitteln eine ausgezeichnete Stabilität auf, so dass es ebenfalls möglich ist, die Salze zusammen mit dem Oxidationsmittel zu konfektionieren.

Eine ebenfalls bevorzugte Ausführungsform zeichnet sich daher dadurch das, dass das anwendungsbereite Farbänderungsmittel durch Vermischen der zwei Komponenten (I) und (II) hergestellt wird, wobei
(I) die ersten Komponente mindestens ein Alkalisierungsmittel sowie gegebenenfalls Oxidationsfarbstoffvorprodukte enthält und
(II) die zweite Komponente Wasserstoffperoxid, Natriumchlorid (a) und Kaliumchlorid (b) enthält.

Die optional enthaltenen Salze aus der Gruppe (c) können in der Komponente (I) und/oder in der Komponente (II) enthalten sein.

Wie bereits zuvor beschrieben sind die oxidativen Farbveränderungsmittel, welche Natriumchlorid (a) in bestimmten Mengenbereichen enthalten, besonders geeignet zur Vermeidung des oxidativen Abbaus der Peptidstruktur der Keratinfasern.

Aus diesem Grund ist ein besonders bevorzugtes anwendungsbereites Mittel dadurch gekennzeichnet, dass es Natriumchlorid (a) in einer Menge von 0,05 bis 3,5 Gew.-%, bevorzugt von 0,1 bis 2,5 Gew.-%, weiter bevorzugt von 0,2 bis 1,5 Gew.-% und besonders bevorzugt von 0,3 bis 0,8 Gew.-%, bezogen auf das Gesamtgewicht des anwendungsbereiten Mittels, enthält.

In einer explizit ganz besonders bevorzugten Ausführungsform enthalten die erfindungsgemäßen Mittel Natriumchlorid und Kaliumchlorid in bestimmten Gewichtsverhältnissen.

Aus diesem Grund ist eine ganz besonders bevorzugtes erfindungsgemäßes Mittel dadurch gekennzeichnet, dass es Natriumchlorid (a) und Kaliumchlorid (b) in einem Gewichtsverhältnis (a)/(b) von 20:1 bis 1:1, bevorzugt von 18:1 bis 3:1, weiter bevorzugt von 16:1 bis 6:1 und besonders bevorzugt von 14:1 bis 9:1, bezogen auf das Gesamtgewicht des anwendungsbereiten Mittels, enthält. Beispielsweise enthält ein bevorzugtes anwendungsbereites Mittel, bezogen auf das Gesamtgewicht des anwendungsbereiten Mittels,
(a) 0,05 bis 3,5 Gew.-% Natriumchlorid
(b) Kaliumchlorid und
(d) Wasserstoffperoxid,
wobei Natriumchlorid (a) und Kaliumchlorid (b) in einem Gewichtsverhältnis (a)/(b) von 20:1 bis 1:1 enthalten sind.

Ein weiteres besonders bevorzugtes anwendungsbereites Mittel enthält, bezogen auf das Gesamtgewicht des anwendungsbereiten Mittels,
(a) 0,05 bis 3,5 Gew.-% Natriumchlorid
(b) Kaliumchlorid und
(d) Wasserstoffperoxid,
wobei Natriumchlorid (a) und Kaliumchlorid (b) in einem Gewichtsverhältnis (a)/(b) von 18:1 bis 3:1 enthalten sind.

Ein weiteres besonders bevorzugtes anwendungsbereites Mittel enthält, bezogen auf das Gesamtgewicht des anwendungsbereiten Mittels,
(a) 0,05 bis 3,5 Gew.-% Natriumchlorid
(b) Kaliumchlorid und
(d) Wasserstoffperoxid,
wobei Natriumchlorid (a) und Kaliumchlorid (b) in einem Gewichtsverhältnis (a)/(b) von 16:1 bis 6:1 enthalten sind.

Ein weiteres besonders bevorzugtes anwendungsbereites Mittel enthält, bezogen auf das Gesamtgewicht des anwendungsbereiten Mittels,
(a) 0,05 bis 3,5 Gew.-% Natriumchlorid
(b) Kaliumchlorid und
(d) Wasserstoffperoxid,
wobei Natriumchlorid (a) und Kaliumchlorid (b) in einem Gewichtsverhältnis (a)/(b) von 14:1 bis 9:1 enthalten sind.

Der Verbraucher muss den Färbe- bzw. Blondierprozess normalerweise in regelmäßigen Zeitabständen wiederholen. Spätestens wenn das Haar nachgewachsen ist und im Ansatzbereich das Haares Farbunterschiede sichtbar werden, muss eine Ansatzbehandlung erfolgen, bei welcher in der Regel auch die bereits farbveränderten Längen des Haares nochmals behandelt werden. Wünscht der Verbraucher sich eine besonders starke Aufhellung des Haares, kann er auch das komplette Haar in kurzen Zeitabständen wiederholt mit dem oxidativen Farbveränderungsmittel behandeln.

Es hat sich herausgestellt, dass die oxidativen Veränderungen der keratinischen Fasern auch bei mehrfacher, innerhalb von kurzen Zeitabständen wiederholter Applikationen minimiert werden können, wenn Natriumchlorid (a) und Wasserstoffperoxid (d) in einem aufeinander abgestimmten Gewichtsverhältnis eingesetzt werden.

Ein weiteres erfindungsgemäß ganz besonders bevorzugtes Mittel ist daher dadurch gekennzeichnet, dass es Natriumchlorid (a) und Wasserstoffperoxid (d) in einem Gewichtsverhältnis (a)/(d) von 2:1 bis 1:6, bevorzugt von 1:1 bis 1:5, weiter bevorzugt von 1:2 bis 1:4 und besonders bevorzugt von 1:3, bezogen auf das Gesamtgewicht des anwendungsbereiten Mittels, enthält.

Auch die Anwesenheit weiterer Mineralsalze hat sich im Hinblick auf eine möglichst geringe oxidative Veränderung der keratinischen Peptidketten als vorteilhaft herausgestellt.

Ein erfindungsgemäß ebenfalls besonders bevorzugtes Mittel zeichnet sich daher dadurch aus, dass es zusätzlich mindestens eine Kombination aus zwei verschiedenen Salzen der Gruppe (c) enthält, die ausgewählt ist aus Calciumchlorid/Magnesiumcarbonat, Calciumchlorid/Magnesiumhydrogencarbonat, Calciumchlorid/Magnesiumsulfat, Calciumchlorid/Magnesiumhydrogensulfat, Calciumchlorid/ Magnesiumchlorid, Calciumchlorid/Calciumcarbonat, Calciumchlorid/Calciumhydrogencarbonat, Calciumchlorid/Calciumhydrogensulfat und/oder Calciumchlorid/Calciumsulfat. Beispielsweise enthält ein bevorzugtes anwendungsbereites Mittel
(a) Natriumchlorid
(b) Kaliumchlorid
(c) Magnesiumcarbonat und
(d) Wasserstoffperoxid.

Ein weiteres bevorzugtes anwendungsbereites Mittel enthält
(a) Natriumchlorid
(b) Kaliumchlorid
(c) Magnesiumhydrogencarbonat und
(d) Wasserstoffperoxid.

Ein weiteres bevorzugtes anwendungsbereites Mittel enthält
(a) Natriumchlorid
(b) Kaliumchlorid
(c) Magnesiumsulfat und
(d) Wasserstoffperoxid.

Ein weiteres bevorzugtes anwendungsbereites Mittel enthält
(a) Natriumchlorid
(b) Kaliumchlorid
(c) Magnesiumhydrogensulfat
(d) Wasserstoffperoxid.

Ein weiteres bevorzugtes anwendungsbereites Mittel enthält
(a) Natriumchlorid
(b) Kaliumchlorid
(c) Magnesiumchlorid und
(d) Wasserstoffperoxid.

Ein bevorzugtes anwendungsbereites Mittel enthält
(a) Natriumchlorid
(b) Kaliumchlorid
(c) Calciumcarbonat und
(d) Wasserstoffperoxid.

Ein bevorzugtes anwendungsbereites Mittel enthält
(a) Natriumchlorid
(b) Kaliumchlorid
(c) Calciumhydrogencarbonat und
(d) Wasserstoffperoxid.

Ein bevorzugtes anwendungsbereites Mittel enthält
(a) Natriumchlorid
(b) Kaliumchlorid
(c) Calciumhydrogensulfat und
(d) Wasserstoffperoxid.

Ein bevorzugtes anwendungsbereites Mittel enthält
(a) Natriumchlorid
(b) Kaliumchlorid
(c) Calciumsulfat und
(d) Wasserstoffperoxid.

Ein bevorzugtes anwendungsbereites Mittel enthält
(a) Natriumchlorid
(b) Kaliumchlorid
(c) Calciumchlorid und
(d) Wasserstoffperoxid.

Ein weiteres bevorzugtes anwendungsbereites Mittel
(a) Natriumchlorid
(b) Kaliumchlorid
(c) Calciumchlorid/Magnesiumcarbonat und
(d) Wasserstoffperoxid.

Ein weiteres bevorzugtes anwendungsbereites Mittel enthält
(a) Natriumchlorid
(b) Kaliumchlorid
(c) Calciumchlorid/Magnesiumhydrogencarbonat und
(d) Wasserstoffperoxid.

Ein weiteres bevorzugtes anwendungsbereites Mittel enthält
(a) Natriumchlorid
(b) Kaliumchlorid
(c) Calciumchlorid/Magnesiumsulfat und
(d) Wasserstoffperoxid.

Ein weiteres bevorzugtes anwendungsbereites Mittel enthält
(a) Natriumchlorid
(b) Kaliumchlorid
(c) Calciumchlorid/Magnesiumhydrogensulfat und
(d) Wasserstoffperoxid.

Ein weiteres bevorzugtes anwendungsbereites Mittel enthält
(a) Natriumchlorid
(b) Kaliumchlorid
(c) Calciumchlorid/ Magnesiumchlorid und
(d) Wasserstoffperoxid.

Ein weiteres bevorzugtes anwendungsbereites Mittel enthält
(a) Natriumchlorid
(b) Kaliumchlorid
(c) Calciumchlorid/Calciumcarbonat und
(d) Wasserstoffperoxid.

Ein weiteres bevorzugtes anwendungsbereites Mittel enthält
(a) Natriumchlorid
(b) Kaliumchlorid
(c) Calciumchlorid/Calciumhydrogencarbonat und
(d) Wasserstoffperoxid.

Ein weiteres bevorzugtes anwendungsbereites Mittel enthält
(a) Natriumchlorid
(b) Kaliumchlorid
(c) Calciumchlorid/Calciumhydrogensulfat und
(d) Wasserstoffperoxid.

Ein weiteres bevorzugtes anwendungsbereites Mittel enthält
(a) Natriumchlorid
(b) Kaliumchlorid
(c) Calciumchlorid/Calciumsulfat und
(d) Wasserstoffperoxid.

Die anwendungsbereiten oxidativen Farbänderungsmittel können weiterhin zusätzliche Wirk-, Hilfs- und Zusatzstoffe enthalten, um die Färbe- bzw. Aufhellleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Erfindungsgemäß geeignete Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül.

Erfindungsgemäß geeignete Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß geeignete Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Die anwendungsbereiten Farbveränderungsmittel können auch mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Geeignete Verdickungsmittel sind anionische, synthetische Polymere, kationische, synthetische Polymere, natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen, nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit. Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 7 und 11, insbesondere zwischen 8 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, welches ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Bezüglich weiterer bevorzugter Ausführungsformen des erfindungsgemäßen anwendungsbereiten Mittels gilt mutatis mutandis das zur erfindungsgemäßen Verwendung Gesagte. Die zuvor beschriebenen erfindungsgemäßen Mittel wirken sich insbesondere bei mehrfacher Applikation vorteilhaft auf die mechanischen Eigenschaften und die Reißfestigkeit der behandelten Keratinfasern aus. Ein weiterer Gegenstand der vorliegenden Erfindung ist daher ein Verfahren zur wiederholten, haarschonenden oxidativen Färbung und/oder zur wiederholten, haarschonenden oxidativen Aufhellung von keratinischen Fasern, umfassend die Schritte
(A) Applikation eines zuvor beschriebenen, erfindungsgemäßen Mittels auf die Fasern,
(B) Einwirken lassen des in Schritt (A) applizierten Mittels für einen Zeitraum von 5 bis 45 Minuten auf den Fasern, dann
(C) Abspülen des Mittels von den Fasern,
wobei das die Schritte (A) bis (C) umfassende Verfahren auf den keratinischen Fasern innerhalb eines Zeitraums von 8 Wochen wiederholt, d.h. mindestens zweimal, bevorzugt mindestens dreimal, durchgeführt wird.

Bezüglich weiterer bevorzugter Ausführungsformen des erfindungsgemäßen Verfahrens gilt mutatis mutandis das zu der erfindungsgemäßen Verwendung und den erfindungsgemäßen Mitteln Gesagte. Der Cysteinsäuregehalt von Keratinfasern bzw. Haarsträhnen kann mittels Nahinfrarotspektroskopie (NIR-Spektroskopie) bestimmt werden. Die NIR-Spektroskopie erlaubt die direkte Quantifizierung des Cysteinsäuregehaltes, ohne das Haar durch eine Analyse in seiner Struktur zu verändern oder zu zerstören (vgl. auch Y. Miyamae et al. IFSCC Magazine, 9, 219 (2006) und Y. Miyamae et al., Appl. Spectroscopy , 61, (2) 212 (2007)). Hierbei wird das Haar mit Infrarotstrahlung (Wärmestrahlung im Nahinfrarotbereich) bestrahlt. Auf diese Weise nicht nur die Oberfläche der Haare analysiert wird, sondern die NIR-Strahlung durchdringt auch die Haare aufgrund des kleinen Absorptionsquerschnittes. Die Strahlung regt die Bestandteile des Haares zu Schwingungen an, wobei die Strahlung definierter Wellenlängen absorbiert wird. Cysteinsäure führt zu charakterisischen Absorptionen im NIR-Spektrum. Anhand der Stärke dieser Absorptionen kann der Gehalt an Cysteinsäure in der Keratinfaser bestimmt werden.

### Beispiele

### 1. Herstellung der Färbemittel

Es wurden die folgenden Farbcremes hergestellt (alle Mengenangaben in Gew.-%)

| **Farbcremes** | **V (Vergleich)** | **E (erfindungsgemäß)** |
|---|---|---|
| Carbomer | 1,00 | 1,00 |
| Vitamin C | 0,20 | 0,20 |
| Cetearylalkokohol (C₁₆/C₁₈-Fettalkohol) | 2,00 | 2,00 |
| PEG-40 Castor Oil | 0,38 | 0,38 |
| Natrium Cetearylsulfat | 0,125 | 0,125 |
| Dinatrium Cocoamphodipropionat | 4,20 | 4,20 |
| Monoethanolamin | 5,00 | 5,00 |
| Xiameter OFX-0193 Fluid | 1,00 | 1,00 |
| (INCI: PEG-12 Dimethicone) | | |
| Natriumsulfit | 0,20 | 0,20 |
| p-Toluylendiamin, Sulfat | 3,26 | 3,26 |
| Resorcin | 0,65 | 0,65 |
| 4-Chlorresorcin | 0,71 | 0,71 |
| m-Aminophenol | 0,15 | 0,15 |
| p-Amino-o-cresol (5-Amino-2-methylphenol) | 0,08 | 0,08 |
| 3-Amino-2-methylamino-6-methoxypyridin | 0,22 | 0,22 |
| 1-Hydroxyethan-1,1-diphosphonsäure | 0,12 | 0,12 |
| Taurin | 2,00 | --- |
| Coffein | 0,50 | -- |
| Mineralsalze La Toja* | --- | 1,00 |
| Parfum | 0,60 | 0,60 |
| Wasser | ad 100 | ad 100 |
| | | |

| | | |
|---|---|---|
| *Mineralsalzgemisch "La Toja" Zusammensetzung: Natriumchlorid: 78,2 Gew.-% Kaliumchlorid: 7,01 Gew.-% Calciumchlorid: 8,40 Gew.-% Magnesiumchlorid: 1,64 Gew.-% Calciumsulfat: 1,59 Gew.-% weitere Salze: ad 100 Gew.-% | | |

Die Farbcremes V und E wurden jeweils im Gewichtsverhältnis 1:1 mit der folgenden Oxidationsmittelzubereitung vermischt.

| **Oxidationsmittelzubereitung** | |
|---|---|
| EDTA (Ethylendiamintetaessigsäure), Dinatriumsalz | 0,15 |
| Dinatriumpyrophosphat | 0,30 |
| Natriumbenzoat | 0,04 |
| Cetearylalkocohol | 1,68 |
| PEG-40 Castor Oil | 0,32 |
| Natrium Cetearylsulfat | 0,10 |
| Wasserstoffperoxid (50 %ige wässrige Lösung | 6,00 |
| Phosphosäure | 0,04 |
| Wasser | ad 100 |

### 2. Applikation

Die auf diese Weise hergestellten anwendungsbereiten oxidativen Färbemittel wurden auf jeweils fünf Haarsträhnen (Kerling, Euronaturhaar, 6-0) appliziert, für 10 Minuten einwirken gelassen und wieder abgespült. Danach wurden die Haarsträhnen getrocknet. Auf jeder Haarsträhne wurde die Anwendung dreimal wiederholt.

### 3. Messung des Cysteinsäuregehaltes

Zur Messung der durch die dreimalige Anwendung hervorgerufenen Haarschädigung wurde der Cysteinsäurewert jeder behandelten Haarsträhne mittels quantitativer NIR-Spektroskopie bestimmt. Die Aufnahme der Spektren erfolgte mit einem MPA™ ^{FT}-NIR Spetkrometer der Firma Bruker Optik GmbH. Der Infrarot-Bereich umfass den Wellenzahlbereich von 12500 cm⁻¹ bis 4000 cm⁻¹ und ist für Oberton- und Kombinationsschwingungen von z.B. CH-, OH- und NH-Gruppen charakteristisch.

Die Messung der Proben wurde mit dem Intetrationskugelmodul an sechs verschiedenen Probenpositionen in diffuser Reflexion durchgeführt. Für die Analyse der gemessenen NIR-Spektren wurde der Wellenzahlbereich von 7300 cm⁻¹ bis 4020 cm⁻¹gewählt.

Die NIR Spektren von Cystin zeigen im Wellenzahlbereich von 6200 cm⁻¹ bis 5500 cm⁻¹ charakteristische Absorptionsbanden. Verändert sich das Haar durch stärkere Schädigung (d.h. nimmt der Cysteinsäuregehalt in den Haaren zu) wirkt sich dies im NIR-Spektrum auf die für Cysteinsäure charakteristischen Banden bei 5020 cm⁻¹ bis 4020 cm⁻¹ aus. Die quantitative Auswertung der NIR-Spektren erfolgte rechnergestützt.

**NIR-Analysenwert [mol Cysteinsäure / 100 mol Aminosäure]**

| Anwendungsmischung | Haarsträhne 1 | Haarsträhne 2 | Haarsträhne 3 | Haarsträhne 4 | Haarsträhne 5 | Mittelwert |
|---|---|---|---|---|---|---|
| unbehandelt | 0,8 | 1,0 | 0,7 | 0,9 | 0,7 | 0,8 |
| V 3 x gefärbt | 1,3 | 1,1 | 1,3 | 1,1 | 1,1 | 1,2 |
| E 3 x gefärbt | 0,9 | 1,1 | 1,2 | 0,9 | 0,9 | 1,0 |

Haarsträhnen, die dreimal mit dem erfindungsgemäßen oxidativen Färbemittel (E) behandelt wurden, wiesen im Vergleich zur Vergleichsformulierung (V) einen signifikant verringerten Cysteinsäurewert auf.

## Patentansprüche

1. Kosmetische, nicht therapeutische Verwendung von
(a) Natriumchlorid
in Mitteln zum oxidativen Färben und/oder oxidativen Aufhellen von keratinischen Fasern zur Reduzierung der durch die Anwendung der Mittel entstandenen Schädigungen der keratinischen Fasern, **dadurch gekennzeichnet,**
- **dass** die Mittel als Oxidationsmittel (d) Wasserstoffperoxid enthalten und
- **dass** die Mittel Natriumchlorid (ä) und Wasserstoffperoxid (d) in einem Gewichtsverhältnis (a)/(d) von 2:1 bis 1:10, bezogen auf das Gesamtgewicht der anwendungsbereiten Mittel, enthalten.

2. Verwendung nach Anspruch 1, **dadurch gekennzeichnet, dass** die Mittel zusätzlich
(b) Kaliumchlorid
enthalten.

3. Verwendung nach einem der Ansprüche 1 oder 2, **dadurch gekennzeichnet, dass** die Mittel Natriumchlorid (a) in einer Menge von 0,05 bis 3,5 Gew.-%, bevorzugt von 0,1 bis 2,5 Gew.-%, weiter bevorzugt von 0,2 bis 1,5 Gew.-% und besonders bevorzugt von 0,3 bis 0,8 Gew.-%, bezogen auf das Gesamtgewicht der anwendungsbereiten Mittel, enthalten.

4. Verwendung nach einem der Ansprüche 1 bis 3, **dadurch gekennzeichnet, dass** die Mittel Kaliumchlorid (b) in einer Menge von 0,01 bis 1,0 Gew.-%, bevorzugt von 0,02 bis 0,5 Gew.-%, weiter bevorzugt von 0,03 bis 0,3 Gew.-% und besonders bevorzugt von 0,04 bis 0,1 Gew.-%, bezogen auf das Gesamtgewicht der anwendungsbereiten Mittel, enthalten.

5. Verwendung nach einem der Ansprüche 1 bis 4, **dadurch gekennzeichnet, dass** die Mittel Natriumchlorid (a) und Kaliumchlorid (b) in einem Gewichtsverhältnis (a)/(b) von 20:1 bis 1:1, bevorzugt von 18:1 bis 3:1, weiter bevorzugt von 16:1 bis 6:1 und besonders bevorzugt von 14:1 bis 9:1, bezogen auf das Gesamtgewicht der anwendungsbereiten Mittel, enthalten.

6. Verwendung nach einem der Ansprüche 1 bis 5, **dadurch gekennzeichnet, dass** die Mittel zusätzlich mindestens ein weiteres Salz (c) aus der Gruppe Magnesiumcarbonat, Magnesiumhydrogencarbonat, Magnesiumsulfat, Magnesiumhydrogensulfat, Magnesiumchlorid, Calciumcarbonat, Calciumhydrogencarbonat, Calciumhydrogensulfat, Calciumsulfat und/oder Calciumchlorid enthalten.

7. Verwendung nach einem der Ansprüche 1 bis 6, **dadurch gekennzeichnet, dass** die Mittel mindestens eine Kombination aus zwei verschiedenen Salzen der Gruppe (c) enthalten, die ausgewählt ist aus Calciumchlorid/Magnesiumcarbonat, Calciumchlorid/Magnesiumhydrogencarbonat, Calciumchlorid/Magnesiumsulfat, Calciumchlorid/Magnesiumhydrogensulfat, Calciumchlorid/Magnesiumchlorid, Calciumchlorid/Calciumcarbonat, Calciumchlorid/Calciumhydrogencarbonat, Calciumchlorid/Calciumhydrogensulfat und/oder Calciumchlorid/Calciumsulfat.

8. Verwendung nach einem der Ansprüche 1 bis 7, **dadurch gekennzeichnet, dass** die Mittel Natriumchlorid (a) und Wasserstoffperoxid (d) in einem Gewichtsverhältnis (a)/(d) von 1:1 bis 1:8, weiter bevorzugt von 1:2 bis 1:8 und besonders bevorzugt von 1:3 bis 1:6, bezogen auf das Gesamtgewicht der anwendungsbereiten Mittel, enthalten.

9. Anwendungsbereites Mittel zum oxidativen Färben und/oder oxidativen Aufhellen von keratinischen Fasern, enthaltend
(a) Natriumchlorid,
(b) Kaliumchlorid, und
(d) Wasserstoffperoxid,
**dadurch gekennzeichnet, dass** es Natriumchlorid (a) und Wasserstoffperoxid (d) in einem Gewichtsverhältnis (a)/(d) von 2:1 bis 1:6, bezogen auf das Gesamtgewicht des anwendungsbereiten Mittels, enthält.

10. Mittel nach Anspruch 9, **dadurch gekennzeichnet, dass** es (c) mindestens ein weiteres Salz aus der Gruppe Magnesiumcarbonat, Magnesiumhydrogencarbonat, Magnesiumsulfat, Magnesiumhydrogensulfat, Magnesiumchlorid, Calciumcarbonat, Calciumhydrogencarbonat, Calciumhydrogensulfat, Calciumsulfat und/oder Calciumchlorid enthält.

11. Mittel nach einem der Ansprüche 9 bis 10, **dadurch gekennzeichnet, dass** es Natriumchlorid (a) in einer Menge von 0,05 bis 3,5 Gew.-%, bevorzugt von 0,1 bis 2,5 Gew.-%, weiter bevorzugt von 0,2 bis 1,5 Gew.-% und besonders bevorzugt von 0,3 bis 0,8 Gew.-%, bezogen auf das Gesamtgewicht des anwendungsbereiten Mittels, enthält.

12. Mittel nach einem der Ansprüche 9 bis 11 , **dadurch gekennzeichnet, dass** es Natriumchlorid (a) und Kaliumchlorid (b) in einem Gewichtsverhältnis (a)/(b) von 20:1 bis 1:1, bevorzugt von 18:1 bis 3:1, weiter bevorzugt von 16:1 bis 6:1 und besonders bevorzugt von 14:1 bis 9:1, bezogen auf das Gesamtgewicht des anwendungsbereiten Mittels, enthält.

13. Mittel nach einem der Ansprüche 9 bis 12, **dadurch gekennzeichnet, dass** es Natriumchlorid (a) und Wasserstoffperoxid (d) in einem Gewichtsverhältnis (a)/(d) von 1:1 bis 1:5, weiter bevorzugt von 1:2 bis 1:4 und besonders bevorzugt von 1:3, bezogen auf das Gesamtgewicht des anwendungsbereiten Mittels, enthält.

14. Mittel nach einem der Ansprüche 9 bis 13, **dadurch gekennzeichnet, dass** es mindestens eine Kombination aus zwei verschiedenen Salzen der Gruppe (c) enthält, die ausgewählt ist aus Calciumchlorid/Magnesiumcarbonat, Calciumchlorid/Magnesiumhydrogencarbonat, Calciumchlorid/Magnesiumsulfat, Calciumchlorid/Magnesiumhydrogensulfat, Calciumchlorid/Magnesiumchlorid, Calciumchlorid/Calciumcarbonat, Calciumchlorid/Calciumhydrogencarbonat, Calciumchlorid/Calciumhydrogensulfat und/oder Calciumchlorid/Calciumsulfat.

15. Verfahren zur wiederholten, haarschonenden oxidativen Färbung und/oder zur wiederholten, haarschonenden oxidativen Aufhellung von keratinischen Fasern, umfassend die Schritte
(A) Applikation eines Mittels nach einem der Ansprüche 9 bis 14 auf die Fasern,
(B) Einwirkenlassen des in Schritt (A) applizierten Mittels für einen Zeitraum von 5 bis 45 Minuten auf den Fasern, dann
(C) Abspülen des Mittels von den Fasern,
wobei das die Schritte (A) bis (C) umfassende Verfahren auf den keratinischen Fasern innerhalb eines Zeitraums von 8 Wochen wiederholt, d.h. mindestens zweimal, bevorzugt mindestens dreimal, durchgeführt wird.

## Claims

1. The cosmetic, non-therapeutic use of
(a) sodium chloride in agents for oxidative dyeing and/or oxidative lightening of keratin fibers for reducing the damage to the keratin fibers caused by the application of the agents, **characterized**
- **in that** the agents contain hydrogen peroxide as an oxidizing agent (d) and
- **in that** the agents contain sodium chloride (a) and hydrogen peroxide (d) in a weight ratio (a)/(d) of from 2:1 to 1:10, based on the total weight of the ready-to-apply agents.

2. The use according to claim 1, **characterized in that** the agents additionally contain (b) potassium chloride.

3. The use according to one of claims 1 to 2, **characterized in that** the agents contain sodium chloride (a) in an amount of from 0.05 to 3.5 wt.%, preferably from 0.1 to 2.5 wt.%, more preferably from 0.2 to 1.5 wt.% and particularly preferably from 0.3 to 0.8 wt.%, based on the total weight of the ready-to-apply agents.

4. The use according to one of claims 1 to 3, **characterized in that** the agents contain potassium chloride (b) in an amount of from 0.01 to 1.0 wt.%, preferably of from 0.02 to 0.5 wt.%, more preferably of from 0.03 to 0.3 wt.% and particularly preferably of from 0.04 to 0.1 wt.%, based on the total weight of the ready-to-apply agents.

5. The use according to one of claims 1 to 4, **characterized in that** the agents contain sodium chloride (a) and potassium chloride (b) in a weight ratio (a)/(b) of from 20:1 to 1:1, preferably from 18:1 to 3:1, more preferably from 16:1 to 6:1 and particularly preferably from 14:1 to 9:1, based on the total weight of the ready-to-apply agents.

6. The use according to one of claims 1 to 5, **characterized in that** the agents additionally contain at least one further salt (c) from the group of magnesium carbonate, magnesium hydrogen carbonate, magnesium sulfate, magnesium hydrogen sulfate, magnesium chloride, calcium carbonate, calcium hydrogen carbonate, calcium hydrogen sulfate, calcium sulfate and/or calcium chloride.

7. The use according to one of claims 1 to 6, **characterized in that** the agents contain at least one combination of two different salts of group (c), selected from calcium chloride/magnesium carbonate, calcium chloride/magnesium hydrogen carbonate, calcium chloride/magnesium sulfate, calcium chloride/magnesium hydrogen sulfate, calcium chloride/magnesium chloride, calcium chloride/calcium carbonate, calcium chloride/calcium hydrogen carbonate, calcium chloride/calcium hydrogen sulfate and/or calcium chloride/calcium sulfate.

8. The use according to one of claims 1 to 7, **characterized in that** the agents contain sodium chloride (a) and hydrogen peroxide (d) in a weight ratio (a)/(d) of from 1:1 to 1:8, more preferably from 1:2 to 1:8 and particularly preferably from 1:3 to 1:6, based on the total weight of the ready-to-apply agents.

9. A ready-to-apply agent for the oxidative dyeing and/or oxidative lightening of keratin fibers, containing
(a) sodium chloride,
(b) potassium chloride, and
(c) hydrogen peroxide,
**characterized in that** it comprises sodium chloride (a) and hydrogen peroxide (d) in a weight ratio (a)/(d) of from 2:1 to 1:6, based on the total weight of the ready-to-apply agent.

10. The agent according to claim 9, **characterized in that** it (c) contains at least one further salt from the group of magnesium carbonate, magnesium hydrogen carbonate, magnesium sulfate, magnesium hydrogen sulfate, magnesium chloride, calcium carbonate, calcium hydrogen carbonate, calcium hydrogen sulfate, calcium sulfate and/or calcium chloride.

11. The agent according to one of claims 9 to 10, **characterized in that** it contains sodium chloride (a) in an amount of from 0.05 to 3.5 wt.%, preferably from 0.1 to 2.5 wt.%, more preferably from 0.2 to 1.5 wt.% and particularly preferably from 0.3 to 0.8 wt.%, based on the total weight of the ready-to-apply agent.

12. The agent according to one of claims 9 to 11, **characterized in that** it contains sodium chloride (a) and potassium chloride (b) in a weight ratio (a)/(b) of from 20:1 to 1:1, preferably from 18:1 to 3:1, more preferably from 16:1 to 6:1 and particularly preferably from 14:1 to 9:1, based on the total weight of the ready-to-apply agent.

13. The agent according to one of claims 9 to 12, **characterized in that** it contains sodium chloride (a) and hydrogen peroxide (d) in a weight ratio (a)/(d) of from 1:1 to 1:5, more preferably from 1:2 to 1:4 and particularly preferably from 1:3, based on the total weight of the ready-to-apply agent.

14. The agent according to one of claims 9 to 13, **characterized in that** it contains at least one combination of two different salts of group (c), selected from calcium chloride/magnesium carbonate, calcium chloride/magnesium hydrogen carbonate, calcium chloride/magnesium sulfate, calcium chloride/magnesium hydrogen sulfate, calcium chloride/magnesium chloride, calcium chloride/calcium carbonate, calcium chloride/calcium hydrogen carbonate, calcium chloride/calcium hydrogen sulfate and/or calcium chloride/calcium sulfate.

15. A method for the repeated, hair-protecting oxidative dyeing and/or the repeated, hair-protecting oxidative lightening of keratin fibers, comprising the steps of
(A) applying an agent according to one of claims 9 to 14 to the fibers,
(B) allowing the agent applied in step (A) to act on the fibers for a period of from 5 to 45 minutes, then
(C) rinsing the agent off the fibers,
wherein the method comprising steps (A) to (C) is repeated on the keratin fibers within a period of 8 weeks, i.e. is carried out at least twice, preferably at least three times.

## Revendications

1. Utilisation cosmétique, non thérapeutique du
(a) chlorure de sodium dans des agents de teinture oxydante et/ou d'éclaircissement oxydant de fibres kératiniques afin de réduire l'altération de fibres kératiniques par l'application des agents, **caractérisée en ce que**
- les agents contiennent du peroxyde d'hydrogène comme agent oxydant (d) et
- les agents contiennent du chlorure de sodium (ä) et du peroxyde d'hydrogène (d) dans un rapport pondéral (a)/(d) de 2:1 à 1:10, par rapport au poids total des agents prêts à l'emploi.

2. Utilisation selon la revendication 1, **caractérisée en ce que** les agents contiennent en outre
(b) du chlorure de potassium.

3. Utilisation selon l'une des revendications 1 ou 2, **caractérisée en ce que** les agents contiennent du chlorure de sodium (a) en une quantité de 0,05 à 3,5 % en poids, de préférence de 0,1 à 2,5 % en poids, plus préférablement de 0,2 à 1,5 % en poids et de manière particulièrement préférée de 0,3 à 0,8 % en poids, par rapport au poids total des agents prêts à l'emploi.

4. Utilisation selon l'une des revendications 1 ou 3, **caractérisée en ce que** les agents contiennent du chlorure de potassium (b) en une quantité de 0,01 à 1,0 % en poids, de préférence de 0,02 à 0,5 % en poids, plus préférablement de 0,03 à 0,3 % en poids et de manière particulièrement préférée de 0,04 à 0,1 % en poids, par rapport au poids total des agents prêts à l'emploi.

5. Utilisation selon l'une des revendications 1 ou 4, **caractérisée en ce que** les agents contiennent du chlorure de sodium (a) et du chlorure de potassium (b) dans un rapport pondéral (a)/(b) de 20:1 à 1:1, de préférence de 18:1 à 3:1, plus préférablement de 16:1 à 6:1 et de manière particulièrement préférée de 14:1 à 9:1, par rapport au poids total des agents prêts à l'emploi.

6. Utilisation selon l'une des revendications 1 à 5, **caractérisée en ce que** les agents contiennent en outre au moins un autre sel (c) du groupe constitué par le carbonate de magnésium, l'hydrogénocarbonate de magnésium, le sulfate de magnésium, l'hydrogénosulfate de magnésium, le chlorure de magnésium, le carbonate de calcium, l'hydrogénocarbonate de calcium, l'hydrogénosulfate de calcium, le sulfate de calcium et/ou le chlorure de calcium.

7. Utilisation selon les revendications 1 à 6, **caractérisée en ce que** les agents contiennent au moins une combinaison de deux sels différents du groupe (c), laquelle est choisie parmi le chlorure de calcium/carbonate de magnésium, le chlorure de calcium/l'hydrogénocarbonate de magnésium, le chlorure de calcium/sulfate de magnésium, le chlorure de calcium/l'hydrogénocarbonate de magnésium, le chlorure de calcium/chlorure de magnésium, le chlorure de calcium/carbonate de calcium, le chlorure de calcium/l'hydrogénocarbonate de calcium, le chlorure de calcium/l'hydrogénosulfate de calcium et/ou le chlorure de calcium/sulfate de calcium.

8. Utilisation selon l'une des revendications 1 à 7, **caractérisée en ce que** les agents contiennent du chlorure de sodium (a) et du peroxyde d'hydrogène (d) dans un rapport pondéral (a)/(d) de 1:1 à 1:8, plus préférablement de 1:2 à 1:8 et de manière particulièrement préférée de 1:3 à 1:6, par rapport au poids total des agents prêts à l'emploi.

9. Agent prêt à l'emploi pour la teinture oxydante et/ou l'éclaircissement oxydant des fibres kératiniques, contenant
(a) du chlorure de sodium,
(b) du chlorure de potassium, et
(c) du peroxyde d'hydrogène,
**caractérisé en ce qu'**il comprend du chlorure de sodium (a) et du peroxyde d'hydrogène (d) dans un rapport pondéral (a)/(d) de 2:1 à 1:6, par rapport au poids total de l'agent prêt à l'emploi.

10. Agent selon la revendication 9, **caractérisé en ce qu'**il (c) contient au moins un autre sel du groupe constitué par le carbonate de magnésium, l'hydrogénocarbonate de magnésium, le sulfate de magnésium, l'hydrogénosulfate de magnésium, le chlorure de magnésium, le carbonate de calcium, l'hydrogénocarbonate de calcium, l'hydrogénosulfate de calcium, le sulfate de calcium et/ou le chlorure de calcium.

11. Agent selon l'une des revendications 9 à 10, **caractérisé en ce qu'**il contient du chlorure de sodium (a) en une quantité de 0,05 à 3,5 % en poids, de préférence de 0,1 à 2,5 % en poids, plus préférablement de 0,2 à 1,5 % en poids et de manière particulièrement préférée de 0,3 à 0,8 % en poids, par rapport au poids total de l'agent prêt à l'emploi.

12. Agent selon l'une des revendications 9 à 11, **caractérisé en ce qu'**il contient du chlorure de sodium (a) et du chlorure de potassium (b) dans un rapport pondéral (a)/(b) de 20:1 à 1:1, de préférence de 18:1 à 3:1, plus préférablement de 16:1 à 6:1 et de manière particulièrement préférée de 14:1 à 9:1, par rapport au poids total de l'agent prêt à l'emploi.

13. Agent selon l'une des revendications 9 à 12, **caractérisé en ce qu'**il contient du chlorure de sodium (a) et du peroxyde d'hydrogène (d) dans un rapport pondéral (a)/(d) de 1:1 à 1:5, plus préférablement de 1:2 à 1:4 et de manière particulièrement préférée de 1:3, par rapport au poids total de l'agent prêt à l'emploi.

14. Agent selon l'une des revendications 9 à 13, **caractérisé en ce qu'**il contient au moins une combinaison de deux sels différents du groupe (c), choisie parmi le chlorure de calcium/carbonate de magnésium, le chlorure de calcium/l'hydrogénocarbonate de magnésium, le chlorure de calcium/sulfate de magnésium, le chlorure de calcium/l'hydrogénocarbonate de magnésium, le chlorure de calcium/chlorure de magnésium, le chlorure de calcium/carbonate de calcium, le chlorure de calcium/l'hydrogénocarbonate de calcium, le chlorure de calcium/l'hydrogénosulfate de calcium et/ou le chlorure de calcium/sulfate de calcium.

15. Procédé de teinture oxydante répétée, non agressive pour les cheveux et/ou d'éclaircissement oxydant répété, non agressifs pour les cheveux, des fibres kératiniques, comprenant les étapes
(A) d'application d'un agent selon l'une des revendications 9 à 14 sur les fibres ;
(B) consistant à laisser agir l'agent appliqué à l'étape (A) sur les fibres pendant une durée de 5 à 45 minutes, et
(C) de rinçage des fibres pour éliminer l'agent,
le procédé comprenant les étapes (A) à (C) étant répété sur les fibres kératiniques pendant une période de 8 semaines, c'est-à-dire exécuté au moins deux fois, de préférence au moins trois fois.
